(19) Europäisches Patentamt
European Patent Office
Office européen des brevets

(11) Numéro de publication : **0 022 118**
**B1**

(12) **FASCICULE DE BREVET EUROPÉEN**

(45) Date de publication du fascicule du brevet : **01.06.83**

(21) Numéro de dépôt : **80870033.0**

(22) Date de dépôt : **10.06.80**

(51) Int. Cl.³ : **C 07 C143/822**, C 07 D295/12, C 07 D213/74, A 61 K 31/18, A 61 K 31/395// C07D303/36

(54) **Nouveaux dérivés de sulfonyl-aniline, leur procédé de préparation et leur application en thérapeutique.**

(30) Priorité : **14.06.79 FR 7915232**

(43) Date de publication de la demande : **07.01.81 Bulletin 81/01**

(45) Mention de la délivrance du brevet : **01.06.83 Bulletin 83/22**

(84) Etats contractants désignés : **AT BE CH DE FR GB IT LI LU NL SE**

(56) Documents cités :
**FR A 2 353 520**
**FR A 2 390 422**

(73) Titulaire : **SANOFI, société anonyme**
**40, Avenue George V**
**F-75008 Paris (FR)**

(72) Inventeur : **Descamps, Marcel**
**Rue du Bois du Bosquet 30**
**B-1331 Rosieres (BE)**
Inventeur : **Goldenberg, Charles**
**Avenue Vanderaey 155**
**B-1180 Bruxelles (BE)**

(74) Mandataire : **Cauchie, Daniel et al**
**c/o S.A. LABAZ-SANOFI N.V. Avenue De Béjar, 1**
**B-1120 Bruxelles (BE)**

**EP 0 022 118 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Nouveaux dérivés de sulfonyl-aniline, leur procédé de préparation et leur application en thérapeutique

La présente invention se rapporte, d'une manière générale, à de nouveaux dérivés substitués d'aniline, à leur procédé de préparation, à ces mêmes dérivés en tant que médicaments et aux compositions pharmaceutiques les contenant.

L'invention se rapporte plus particulièrement aux composés de formule générale :

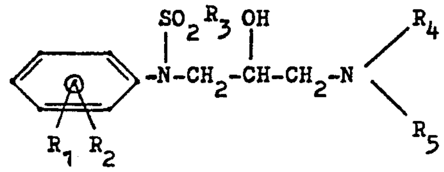

(I)

dans laquelle $R_1$ et $R_2$, qui sont identiques ou différents, représentent un groupement allyloxy, acétamido ou carboxamido, ou un atome d'hydrogène ou de chlore, $R_3$ est un groupement méthyle, phényle, méthylphényle ou méthoxyphényle, $R_4$ représente un atome d'hydrogène et $R_5$ un groupement isopropyle, terbutyle, phénoxy-2 éthyle, phényl-3 propyle, ou $R_4$ et $R_5$ forment avec l'atome d'azote un hétérocycle tel que pyrrolidine, morpholine ou une pipérazine substituée de formule :

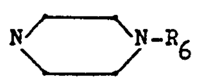

(II)

dans laquelle $R_6$ représente un groupement alkyle linéaire contenant de 1 à 3 atomes de carbone, un radical hydroxy-2 éthyle, phényle, phényle substitué par un atome d'halogène ou par un groupement méthoxy, le groupement benzyle ou pyridyle-2.

L'invention se rapporte également aux sels d'addition d'acide pharmaceutiquement acceptables des composés de formule I.

Les composés de formule I peuvent, d'une manière générale, être préparés par aminolyse, à l'aide d'une amine de formule générale :

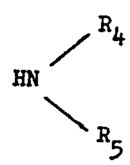

(III)

dans laquelle $R_4$ et $R_5$ ont les mêmes significations que ci-dessus, d'un composé de formule générale :

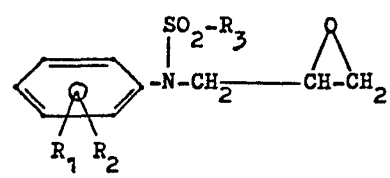

(IV)

dans laquelle $R_1$, $R_2$ et $R_3$ ont les mêmes significations que ci-dessus.

L'opération d'aminolyse s'effectue dans un solvant alcoolique tel que, par exemple l'éthanol.

Les amines de formule générale III sont des produits commerciaux largement disponibles.

Les époxydes de formule IV sont préparées par condensation alcaline d'une sulfonamide de formule :

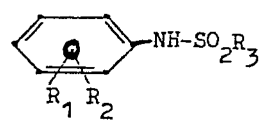

(V)

dans laquelle $R_1$, $R_2$ et $R_3$ ont les mêmes significations que ci-dessus, avec une épihalohydrine telle que, par exemple, l'épichlorhydrine. Les sulfamides de formule V sont préparées par condensation d'une aniline substituée de formule :

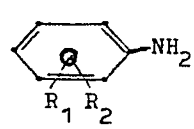

(VI)

dans laquelle $R_1$ et $R_2$ ont les mêmes significations que ci-dessus, avec un chlorure de sulfonyle de formule :

$$Cl—SO_2R_3 \quad (VII)$$

dans laquelle $R_3$ a les mêmes significations que ci-dessus.

Les anilines de formule VI sont soit des produits commerciaux, soit ont été décrites par G.D. TIFFANY dans J. Am. Chem. Soc., 1948, *70,* 592 et par J. DEGUTIS et D. SUKELIENE dans Zh. Obshch. Khim. 1961, *31,* 3326.

Les chlorures de sulfonyle de formule VII sont des produits commerciaux. On a découvert que les composés de l'invention possèdent des propriétés pharmacologiques utiles, susceptibles de les rendre très intéressants pour le traitement des troubles pathologiques du cœur. Plus particulièrement, on a découvert qu'ils possédaient des propriétés susceptibles de les rendre très utiles pour le traitement de l'angine de poitrine.

En conséquence, un autre objet de l'invention se rapporte aux composés de formule I et à leurs sels d'addition non toxiques en tant qu'agents utiles pour le traitement de l'angine de poitrine.

C'est un fait bien connu que les troubles pathologiques du cœur sont très difficiles à maîtriser.

Ceci est particulièrement vrai pour l'angine de poitrine, essentiellement parce qu'il y a de nombreux facteurs qui peuvent déclencher une crise d'angor.

Ce grand nombre de facteurs justifie la conception actuelle de la thérapeutique de l'angor, selon laquelle la valeur d'un principe actif sera directement fonction de la polyvalence de son spectre pharmacologique.

On a découvert que les composés selon l'invention provoquent une bradycardie et diminuent la pression artérielle, composantes favorables à un meilleur rendement énergétique du myocarde. On a également découvert que les composés de l'invention présentent des propriétés antiadrénergiques très intéressantes, non seulement au niveau du système α mais également du système β. Ces dernières propriétés soustraient le cœur à l'action hypermétabolisante des catécholamines et par là concourent aussi à améliorer le rendement cardiaque.

L'étude pharmacologique a été réalisée sur chien anesthésié au pentobarbital sodique (30 mg/kg I.V.) et ayant reçu une dose intraveineuse de 1 mg/kg de sulfate d'atropine.

La substance à étudier est injectée en deux minutes par voie intraveineuse et l'on a en premier lieu suivi l'évolution dans le temps de la fréquence cardiaque et de la pression artérielle et puis l'on a recherché d'éventuelles propriétés antiadrénergiques.

Les composés ci-dessous ont été testés, le plus souvent sous forme de sels d'addition d'acide pharmaceutiquement acceptables.

| Composés | n° code |
|---|---|
| Allyloxy-2 N-[tert. butylamino-3 hydroxy-2 propyl] N-(méthoxy-4 benzènesulfonyl) aniline | 14 |
| Allyloxy-2 N-[((fluoro-4 phényl)-4 pipérazinyl-1)-3 hydroxy-2 propyl] N-méthylsulfonyl aniline | 23 |
| Allyloxy-2 N-[hydroxy-2((méthoxy-2 phényl)-4 pipérazinyl-1)-3 propyl] N-méthylsulfonyl aniline | 30 |
| Allyloxy-2 N-[(benzyl-4 pipérazinyl-1)-3 hydroxy-2 propyl] N-(méthyl-4 benzènesulfonyl) aniline | 40 |
| Allyloxy-2 N-[hydroxy-2((pyridyl-2)-4 pipérazinyl-1)-3 propyl] N-(méthoxy-4 benzènesulfonyl) aniline | 53 |
| Allyloxy-2 N-[hydroxy-2((fluoro-2 phényl)-4 pipérazinyl-1)-3 propyl] N-(méthoxy-4 benzènesulfonyl) aniline | 57 |
| Dichloro-3,4 N-[((chloro-2 phényl)-4 pipérazinyl-1)-3 hydroxy-2 propyl] N-méthylsulfonyl aniline | 75 |
| Dichloro-3,4 N-[hydroxy-2((méthoxy-2 phényl)-4 pipérazinyl-1)-3 propyl] N-méthylsulfonyl aniline | 77 |
| Dichloro-3,4 N-[((chloro-4 phényl-4 pipérazinyl-1)-3 hydroxy-2 propyl] N-(méthoxy-4 benzènesulfonyl) aniline | 84 |
| Acétylamino-4 N-[hydroxy-2(phényl-4 pipérazinyl-1)-3 propyl] N-méthylsulfonyl aniline | 88 |
| Acétylamino-4 N-[hydroxy-2((méthoxy-2 phényl)-4 pipérazinyl-1)-3 hydroxy-2 propyl] N-méthylsulfonyl aniline | 90 |
| Acétylamino-4 N-[hydroxy-2(phényl-4 pipérazinyl-1)-3 propyl] N-(méthyl-4 benzènesulfonyl) aniline | 91 |
| Acétylamino-4 N-[hydroxy-2((méthoxy-2 phényl)-4 pipérazinyl-1)-3 propyl] N-(méthyl-4 benzènesulfonyl) aniline | 94 |
| Acétylamino-4 N-[((chloro-2 phényl)-4 pipérazinyl-1)-3 hydroxy-2 propyl] N-(méthyl-4 benzènesulfonyl) aniline | 95 |
| N-[((chloro-4 phényl)-4 pipérazinyl-1)-3 hydroxy-2 propyl] N-méthylsulfonyl amino-4 phénylacétamide | 99 |
| N-[hydroxy-2((méthoxy-2 phényl)-4 pipérazinyl-1)-3 propyl] N-méthylsulfonyl amino-4 phénylacétamide | 102 |
| N-[hydroxy-2((méthoxy-2 phényl)-4 pipérazino-1)-3 propyl] N-(méthyl-4 benzènesulfonyl) | |

amino-4 phénylacétamide 105

On a observé :

1. Evolution de la fréquence cardiaque

A la dose de 10 mg/kg, les composés n$^{os}$ 14, 40, 57, 77, 84, 91, 94 et 102 provoquent une diminution sensible et durable de la fréquence cardiaque.

2. Evolution de la pression artérielle

A la même dose de 10 mg/kg, les composés n$^{os}$ 23, 30, 40, 53, 75, 77, 88, 90, 91, 94, 95, 102 et 105 provoquent une diminution nette et constante de la pression artérielle.

3. Propriétés anti-adrénergiques

On a tout d'abord déterminé l'effet inhibiteur sur le système $\alpha$ des composés de l'invention, et ce en déterminant la réduction par lesdits composés de l'hypertension artérielle induite par l'épinéphrine.

On administre à un chien anesthésié au pentobarbital sodique et ayant reçu une dose intraveineuse de 1 mg/kg de sulfate d'atropine, une dose d'épinéphrine suffisante pour provoquer une hypertension stable chez l'animal. On répète plusieurs fois l'opération, on note la tension artérielle atteinte et, lorsque celle-ci est revenue à la normale, on administre à l'animal une dose intraveineuse de 10 mg/kg du composé à étudier puis la même dose que ci-dessus d'épinéphrine et l'on observe l'évolution de la pression artérielle au cours du temps.

Les composés n$^{os}$ 14, 23, 30, 40, 53, 57, 75, 77, 84, 88, 90, 91, 94, 99, 102 et 105 inhibent au moins partiellement l'hypertension artérielle induite par l'épinéphrine.

L'inhibition peut être totale et de longue durée dès la dose de 2 mg/kg, par exemple, avec les composés n$^{os}$ 30 et 102.

On a également déterminé l'effet antiadrénergique des composés de l'invention au niveau du système $\beta$, en déterminant la réduction par lesdits composés de la tachycardie induite par l'isoprénaline, en suivant le même mode opératoire que ci-dessus.

Les composés n$^{os}$ 14, 30, 40 et 88 inhibent très nettement la tachycardie induite par l'isoprénaline, montrant ainsi un effet antiadrénergique $\beta$ important. Il est connu que le maximum de l'efficacité thérapeutique d'un médicament dans l'angor est atteint lorsque son effet antiadrénergique $\alpha$ est couplé avec un effet antiadrénergique $\beta$ et également avec un certain effet dépresseur sur la pression artérielle et sur la fréquence cardiaque.

C'est ce qui a été observé pour les composés ci-dessus qui sont les composés préférés de la présente invention.

On a enfin réalisé l'étude de la toxicité aiguë sur le rat, par voie intraveineuse, du composé 40.

On a trouvé une $DL_{50}$, c'est-à-dire une dose tuant la moitié des animaux traités, égale à 12 mg/kg.

Pour l'usage thérapeutique dans le traitement de l'angor, les composés selon l'invention seront administrés, à raison de 100 à 200 mg/jour, sous la forme d'un médicament ou d'une composition pharmaceutique contenant comme principe actif un composé de formule I, ou un sel d'addition d'acide pharmaceutiquement acceptable, associé à un excipient approprié.

Pour l'usage clinique, les composés selon l'invention seront administrés sous la forme de compositions pharmaceutiques convenant au mode d'administration désiré. Ainsi, l'unité d'administration peut prendre la forme, par exemple, d'un comprimé, d'une dragée, d'une capsule, d'une gélule pour l'administration orale, d'une solution pour l'injection ou d'un suppositoire pour l'administration rectale.

Quelle que soit la forme de la composition pharmaceutique, celle-ci comprendra au moins un composé de formule I, ou un de ses sels d'addition d'acide non toxiques en association avec un excipient approprié, ce dernier pouvant être constitué, par exemple, d'au moins un ingrédient sélectionné parmi les substances suivantes : lactose, amidon, talc, stéarate de magnésium, polyvinylpyrrolidone, acide alginique, silice colloïdale ou un agent édulcorant.

L'exemple ci-dessous illustre, de manière non limitative, le procédé de préparation des composés selon l'invention :

Préparation de l'Allyloxy-2 N-[hydroxy-2 isopropylamino-3 propyl] N-méthylsulfonyl aniline

a) Allyloxy-2 N-méthylsulfonyl aniline

Dans un ballon de deux litres à trois cols, muni d'une agitation mécanique, d'une ampoule à brome et d'une mise à l'air, on dissout 149,2 g (1 mole) d'allyloxy-2 aniline dans un mélange de 500 ml de benzène et de 190 ml de pyridine.

On ajoute goutte à goutte à température ambiante une solution de 114,5 g (1 mole) de chlorure de méthane sulfonyle dans 195 ml de diméthylformamide et de 70 ml de benzène.

On agite ensuite à température ambiante pendant 24 heures. On verse le mélange réactionnel dans

l'eau et après une agitation d'une heure, on filtre l'insoluble.

On décante la phase organique et on élimine l'excès de pyridine par un lavage à l'acide chlorhydrique à 20 %.

La phase organique est ensuite lavée deux fois à l'eau puis séchée sur du sulfate de calcium anhydre. On évapore le solvant sous pression réduite et on recristallise le résidu solide dans l'isopropanol. On obtient ainsi 178 g d'allyloxy-2 N-méthylsulfonyl aniline fondant à 90 °C. Rendement : 78,3 %.

De la même manière, mais en partant des produits de départ appropriés, on a également préparé :

| Composés | Point de fusion °C |
|---|---|
| Allyloxy-2 N-(méthyl-4 benzènesulfonyl) aniline | 102 (isopropanol) |
| Allyloxy-2 N-(méthoxy-4 benzènesulfonyl) aniline | 98 (isopropanol) |
| Dichloro-2,6 N-benzènesulfonyl aniline | 156 (éther) |
| Dichloro-2,6 N-(méthyl-4 benzènesulfonyl) aniline | 158 (isopropanol) |
| Dichloro-3,4 N-méthylsulfonyl aniline | 174 (éthanol) |
| Dichloro-3,4 N-(méthyl-4 benzènesulfonyl) aniline | 150 (isopropanol) |
| Dichloro-3,4 N-(méthoxy-4 benzènesulfonyl) aniline | 90 (isopropanol) |
| Acétylamino-4 N-méthylsulfonyl aniline | 208 (méthanol) |
| Acétylamino-4 N-(méthyl-4 benzènesulfonyl) aniline | 184 (éther de pétrole 40/80) |
| N-(méthylsulfonyl) amino-4 phénylacétamide | 206 (méthanol) |
| (N-méthyl-4 benzènesulfonyl) amino-4 phénylacétamide | 162 (isopropanol) |

b) Allyloxy-2 N[(époxy-2,3) propyl] N-méthylsulfonyl aniline

Dans un ballon de 500 ml à trois cols, muni d'une agitation mécanique, d'une ampoule à brome et d'une mise à l'air surmontée d'un tube contenant du chlorure de calcium, on introduit une suspension de 5,1 g d'hydrure de sodium dans 120 ml de diméthylformamide. On y ajoute goutte à goutte une solution de 55 g (0,24 mole) d'allyloxy-2 N-méthylsulfonyl aniline, en refroidissant le milieu réactionnel de façon à ne pas dépasser la température ambiante. On ajoute ensuite en une fois 40 ml d'épichlorhydrine et on maintient l'agitation pendant trois jours à température ambiante. On verse le mélange réactionnel dans l'eau et on l'extrait trois fois avec de l'éther. On lave la phase organique à l'eau et on la sèche sur du sulfate de calcium anhydre.

On évapore ensuite l'éther et on recristallise le résidu solide dans le méthanol. On obtient ainsi 36,5 g d'allyloxy-2 N-[(époxy-2,3) propyl] N-méthylsulfonyl aniline fondant à 78 °C. Rendement : 66,4 %.

De la même manière, mais en partant des produits de départ appropriés, on a également préparé :

| Composés | Point de fusion °C |
|---|---|
| Allyloxy-2 N-[(époxy-2,3) propyl] N-(méthyl-4 benzènesulfonyl) aniline | non analysé |
| Allyloxy-2 N-[(époxy-2,3) propyl] N-(méthoxy-4 benzènesulfonyl) aniline | non analysé |
| Dichloro-2,6 N-[(époxy-2,3) propyl] N-benzènesulfonyl aniline | 126-127 (isopropanol) |
| Dichloro-2,6 N-[(époxy-2,3) propyl] N-(méthyl-4 benzènesulfonyl) aniline | 126 (isopropanol) |
| Dichloro-3,4 N-[(époxy-2,3) propyl] N-méthylsulfonyl aniline | 75 (isopropanol/chloroforme) |
| Dichloro-3,4 N-[(époxy-2,3) propyl] N-(méthyl-4 benzènesulfonyl) aniline | 200 (isopropanol/chloroforme) |
| Dichloro-3,4 N-[(époxy-2,3) propyl] N-(méthoxy-4 benzènesulfonyl) aniline | 110 (isopropanol) |
| Acétylamino-4 N-[(époxy-2,3) propyl] N-méthylsulfonyl aniline | 136-140 (isopropanol) |

| | |
|---|---|
| Acétylamino-4 N-[(époxy-2,3) propyl] N-(méthyl-4 benzènesulfonyl) aniline | 114-115 (isopropanol) |
| N-[(époxy-2,3) propyl] N-méthylsulfonyl amino-4 phénylacétamide | 154-159 (méthanol) |
| N-[(époxy-2,3) propyl] N-(méthyl-4 benzènesulfonyl) amino-4 phénylacétamide | 137-140 (isopropanol) |

c) Allyloxy-2 N-[hydroxy-2 isopropylamino-3 propyl] N-méthylsulfonyl aniline chlorhydrate.

Dans un ballon d'un litre, on dissout 56,7 g (0,2 mole) d'allyloxy-2 N-[(époxy-2,3) propyl] N-méthylsulfonyl aniline et 50 ml d'isopropylamine dans 300 ml d'éthanol absolu. On chauffe le mélange réactionnel au reflux pendant 24 heures puis on élimine l'excès d'isopropylamine et le solvant par évaporation sous vide.

On dissout le résidu dans l'éther et l'on ajoute une solution saturée d'acide chlorhydrique dans l'éther. Le chlorhydrate précipité est filtré et recristallisé dans l'isopropanol.

On obtient ainsi 58,5 g de chlorhydrate d'allyloxy-2 N-[hydroxy-2 isopropylamino-3 propyl] N-méthylsulfonyl aniline fondant à 139-141 °C.

Rendement : 77,4 %.

De la même manière, mais en utilisant les produits de départ appropriés, on a également préparé :

| Composés | Point de fusion °C |
|---|---|
| Allyloxy-2 N-[tert. butylamino-3 hydroxy-2 propyl] N-méthylsulfonylaniline. Chlorhydrate | 183 (isopropanol) |
| Allyloxy-2 N-méthylsulfonyl N-[phénoxy-2 éthyl amino-3 hydroxy-2 propyl] aniline. p. toluène sulfonate | 118-120 (isopropanol) |
| Allyloxy-2 N-méthylsulfonyl N-[hydroxy-2 pyrrolidino-3 propyl] aniline. Oxalate acide | 119-121 (acétate d'éthyle/méthanol) |
| Allyloxy-2 N-méthylsulfonyl N-[hydroxy-2(phényl-3 propyl) amino-3 propyl] aniline. Oxalate acide | 154-156 (acétate d'éthyle/méthanol) |
| Allyloxy-2 N-méthylsulfonyl N-[hydroxy-2 morpholino-3 propyl] aniline. Oxalate acide | 146-151 (isopropanol) |
| Allyloxy-2 N-méthylsulfonyl N-[hydroxy-2(méthyl-4 pipérazinyl-1)-3 propyl] aniline. Dichlorhydrate | 223-229 (isopropanol) |
| Allyloxy-2 N-méthylsulfonyl N-[(éthyl-4 pipérazinyl-1)-3 hydroxy-2 propyl] aniline. Dichlorhydrate | 211-213 (acétone) |
| Allyloxy-2 N-méthylsulfonyl N-[hydroxy-2(propyl-4 pipérazinyl-1)-3 propyl] aniline. Dichlorhydrate | 186-189 (acétone) |
| Allyloxy-2 N-méthylsulfonyl N-[hydroxy-2(phényl-4 pipérazinyl-1)-3 propyl] aniline. Dichlorhydrate | 127-131 (acétone) |
| Allyloxy-2 N-[((fluoro-4 phényl)-4 pipérazinyl-1)-3 hydroxy-2 propyl] N-méthylsulfonyl aniline. Oxalate acide | 174-180 (acétone) |
| Allyloxy-2 N-[((hydroxy-2 éthyl)-4 pipérazino-1)-3 hydroxy-2 propyl] N-méthylsulfonyl aniline | 148-151 (isopropanol) |
| Allyloxy-2 N-[hydroxy-2((pyridyl-2)-4 pipérazinyl-1)-3 propyl] N-méthylsulfonyl aniline. Oxalate neutre | 175-181 (méthanol) |
| Allyloxy-2 N-[((chloro-3 phényl)-4 pipérazinyl-1)-3 hydroxy-2 propyl] N-méthylsulfonyl aniline. Oxalate acide | 150-155 (acétone) |
| Allyloxy-2 N-((chloro-4 phényl)-4 pipérazinyl-1)-3 hydroxy-2 propyl] N-méthylsulfonyl aniline. Oxalate acide | 163-166 (acétone) |
| Allyloxy-2 N-[hydroxy-2((méthoxy-4 phényl)-4 pipérazinyl-1)-3 propyl] N-méthylsulfonyl aniline. Oxalate acide | 116-118 (acétone) |

Allyloxy-2 N-[(benzyl-4 pipérazinyl-1)-3 hydroxy-2 propyl] N-méthylsulfonyl aniline. Difumarate 175-177,5 (isopropanol)

Allyloxy-2 N-[hydroxy-2((méthoxy-2 phényl)-4 pipérazinyl-1)-3 propyl] N-méthylsulfonyl aniline. Chlorhydrate 170-173 (isopropanol)

Allyloxy-2 N-[((fluoro-2 phényl)-4 pipérazinyl-1)-3 hydroxy-2 propyl] N-méthylsulfonyl aniline 159-165 (isopropanol)

Allyloxy-2 N-[hydroxy-2 isopropylamino-3 propyl] N-(méthyl-4 benzène sulfonyl) aniline. Chlorhydrate 165-166 (isopropanol)

Allyloxy-2 N-[tert. butylamino-3 hydroxy-2 propyl] N-(méthyl-4 benzènesulfonyl) aniline. Chlorhydrate 126 (acétate d'éthyle)

Allyloxy-2 N-[hydroxy-2 pyrrolidino-3 propyl] N-(méthyl-4 benzènesulfonyl) aniline. Chlorhydrate 144-146,5 (isopropanol)

Allyloxy-2 N-[hydroxy-2 morpholino-3 propyl] N-(méthyl-4 benzènesulfonyl) aniline. Chlorhydrate 81-84 (isopropanol)

Allyloxy-2 N-[hydroxy-2(phénoxy-2 éthylamino)-3 propyl] N-(méthyl-4 benzènesulfonyl) aniline. Chlorhydrate 143-145 (isopropanol)

Allyloxy-2 N-[hydroxy-2(phényl-3 propylamino)-3 propyl] N-(méthyl-4 benzènesulfonyl) aniline. Oxalate neutre 167-168 (éthanol)

Allyloxy-2 N-[hydroxy-2(méthyl-4 pipérazinyl-1)-3 propyl] N-(méthyl-4 benzènesulfonyl) aniline. Dichlorhydrate 176-185 (isopropanol)

Allyloxy-2 N-[(éthyl-4 pipérazinyl-1)-3 hydroxy-2 propyl] N-(méthyl-4 benzènesulfonyl) aniline. Dichlorhydrate 159-164 (isopropanol)

Allyloxy-2 N-[hydroxy-2((propyl-4 pipérazinyl-1)-3 propyl] N-(méthyl-4 benzènesulfonyl) aniline 144-148 (isopropanol)

Allyloxy-2 N-[hydroxy-2((hydroxy-2 éthyl)-4 pipérazinyl-1)-3 propyl] N-(méthyl-4 benzènesulfonyl) aniline. Dichlorhydrate 157-159 (isopropanol)

Allyloxy-2 N-[((chloro-4 phényl)-4 pipérazinyl-1)-3 hydroxy-2 propyl] N-(méthyl-4 benzènesulfonyl) aniline. Oxalate neutre 176-177 (méthanol)

Allyloxy-2 N-[hydroxy-2((pyridyl-2)-4 pipérazinyl-1)-3 propyl] N-(méthyl-4 benzènesulfonyl) aniline. Oxalate neutre 203-204,5 (méthanol)

Allyloxy-2 N-[hydroxy-2(phényl-4 pipérazinyl-1)-3 propyl] N-(méthyl-4 benzènesulfonyl) aniline. Chlorhydrate 193-203 (méthanol)

Allyloxy-2 N-[((chloro-3 phényl)-4 pipérazinyl-1)-3 hydroxy-2 propyl] N-(méthyl-4 benzènesulfonyl) aniline. Oxalate neutre 177-179 (acétone)

Allyloxy-2 N-[benzyl-4 pipérazinyl-1)-3 hydroxy-2 propyl] N-(méthyl-4 benzènesulfonyl) aniline. Dichlorhydrate 142-146 (acétone)

Allyloxy-2 N-[((fluoro-4 phényl)-4 pipérazinyl-1)-3 hydroxy-2 propyl] N-(méthyl-4 benzènesulfonyl) aniline. Chlorhydrate 191-194 (isopropanol]

Allyloxy-2 N-[hydroxy-2((méthoxy-4 phényl)-4 pipérazinyl-1)-3 propyl] N-(méthyl-4 benzènesulfonyl) aniline. Chlorhydrate 211-214 (méthanol)

Allyloxy-2 N-[hydroxy-2((méthoxy-2 phényl)-4 pipérazinyl-1)-3 propyl] N-(méthyl-4 benzènesulfonyl) aniline. Oxalate neutre 161-165 (isopropanol)

Allyloxy-2 N-[((fluoro-2 phényl)-4 pipérazinyl-1)-3 hydroxy-2 propyl] N-(méthyl-4

benzènesulfonyl) aniline. Chlorhydrate 160-165 (isopropanol)

Allyloxy-2 N-[hydroxy-2 isopropylamino-3 propyl] N-(méthoxy-4 benzènesulfonyl) aniline. Chlorhydrate 137-140 (isopropanol)

Allyloxy-2 N-[tert. butylamino-3 hydroxy-2 propyl] N-(méthoxy-4 benzènesulfonyl) aniline. Chlorhydrate 129-130 (isopropanol)

Allyloxy-2 N-[hydroxy-2(phénoxy-2 éthylamino-3 propyl] N-(méthoxy-4 benzènesulfonyl) aniline. Chlorhydrate 162-164 (acétate d'éthyle)

Allyloxy-2 N-[hydroxy-2(phényl-3 propylamino)-3 propyl] N-(méthoxy-4 benzènesulfonyl) aniline. Oxalate neutre 157-158 (acétate d'éthyle)

Allyloxy-2 N-[hydroxy-2 pyrrolidino-3 propyl] N-(méthoxy-4 benzènesulfonyl) aniline. Chlorhydrate 161-162,5 (acétate d'éthyle)

Allyloxy-2 N-[hydroxy-2 morpholino-3 propyl] N-(méthoxy-4 benzènesulfonyl) aniline. Chlorhydrate 124-127 (isopropanol)

Allyloxy-2 N-[hydroxy-2(méthyl-4 pipérazinyl-1)-3 propyl] N-(méthoxy-4 benzènesulfonyl) aniline. Dichlorhydrate 191-193 (isopropanol)

Allyloxy-2 [(éthyl-4 pipérazinyl-1)-3 hydroxy-2 propyl] N-(méthoxy-4 benzènesulfonyl) aniline. Dichlorhydrate 147-150 (isopropanol)

Allyloxy-2 [hydroxy-2(n-propyl-4 pipérazinyl-1)-3 propyl] N-(méthoxy-4 benzènesulfonyl) aniline. Dichlorhydrate 181-184 (acétone)

Allyloxy-2 N-[hydroxy-2((hydroxy-2 éthyl)-4 pipérazinyl-1)-3 propyl] N-(méthoxy-4 benzènesulfonyl) aniline. Dichlorhydrate 195-197 (méthanol)

Allyloxy-2 N-[hydroxy-2(phényl-4 pipérazinyl-1)-3 propyl] N-(méthoxy-4 benzènesulfonyl) aniline. Chlorhydrate 220-222 (isopropanol)

Allyloxy-2 N-[(benzyl-4 pipérazinyl-1)-3 hydroxy-2 propyl] N-(méthoxy-4 benzènesulfonyl) aniline. Dichlorhydrate 144-147 (acétone)

Allyloxy-2 N-[((chloro-3 phényl)-4 pipérazinyl-1)-3 hydroxy-2 propyl] N-(méthoxy-4 benzènesulfonyl) aniline. Oxalate neutre 171-175 (acétone)

Allyloxy-2 N-[hydroxy-2((méthoxy-4 phényl)-4 pipérazinyl-1)-3 propyl] N-(méthoxy-4 benzènesulfonyl) aniline. Oxalate neutre 161-164 (isopropanol)

Allyloxy-2 N-[hydroxy-2((pyridyl-2)-4 pipérazinyl-1)-3 propyl] N-(méthoxy-4 benzènesulfonyl) aniline. Oxalate neutre 183-185 (acétone)

Allyloxy-2 N-[((chloro-4 phényl)-4 pipérazinyl-1)-3 hydroxy-2 propyl] N-(méthoxy-4 benzènesulfonyl) aniline. Oxalate acide 123-124 (acétone)

Allyloxy-2 N-[((fluoro-4 phényl)-4 pipérazinyl-1)-3 hydroxy-2 propyl] N-(méthoxy-4 benzènesulfonyl) aniline. Oxalate neutre 160-161,5 (méthanol)

Allyloxy-2 N-[hydroxy-2((méthoxy-2 phényl-4 pipérazinyl-1)-3 propyl] N-(méthoxy-4 benzènesulfonyl) aniline 185-188,5 (isopropanol)

Allyloxy-2 N-[hydroxy-2((fluoro-2 phényl)-4 pipérazinyl-1)-3 propyl] N-(méthoxy-4 benzènesulfonyl) aniline 175-179 (isopropanol)

Dichloro-2,6 N-benzènesulfonyl N-[hydroxy-2(méthyl-4 pipérazinyl-1)-3 propyl] aniline. Dichlorhydrate 239-243 (acétone)

Dichloro-2,6 N-benzènesulfonyl N-[hydroxy-2(phényl-4 pipérazinyl-1)-3 propyl] aniline 131-134 (isopropanol)

Dichloro-2,6 N-benzènesulfonyl N-[hydroxy-2((chloro-4 phényl)-4 pipérazinyl-1)-3

propyl] aniline. Oxalate acide 137-143 (acétone)

Dichloro-2,6 N-benzènesulfonyl N-[(benzyl-4 pipérazinyl-1)-3 hydroxy-2 propyl] aniline. Dichlorhydrate 220-228 (acétone)

Dichloro-2,6 N-benzènesulfonyl N-[hydroxy-2((méthoxy-4 phényl)-4 pipérazinyl-1)-3 propyl] aniline. Oxalate acide 131-136 (acétone)

Dichloro-2,6 N-benzènesulfonyl N-[((chloro-3 phényl)-4 pipérazinyl-1)-3 hydroxy-2 propyl] aniline. Oxalate acide 188-189 (méthanol)

Dichloro-2,6 N-benzènesulfonyl N-[hydroxy-2(fluoro-4 phényl-4 pipérazinyl-1)-3 propyl] aniline. Oxalate acide 174-176 (isopropanol)

Dichloro-2,6 N-benzènesulfonyl N-[hydroxy-2((méthoxy-2 phényl)-4 pipérazinyl-1)-3 propyl] aniline. Oxalate acide 196-197 (isopropanol)

Dichloro-2,6 N-[hydroxy-2(méthyl-4 pipérazinyl-1)-3 propyl] N-(méthyl-4 benzènesulfonyl) aniline. Dichlorhydrate 230-234 (acétone)

Dichloro-2,6 N-[hydroxy-2((méthoxy-4 phényl)-4 pipérazinyl-1)-3 propyl] N-(méthyl-4 benzènesulfonyl) aniline. Oxalate neutre 164-169 (acétone)

Dichloro-2,6 N-[hydroxy-2(phényl-4 pipérazinyl-1)-3 propyl] N-(méthyl-4 benzènesulfonyl) aniline. Oxalate acide 230-234 (acétone)

Dichloro-2,6 N-[(benzyl-4 pipérazinyl-1)-3 hydroxy-2 propyl] N-(méthyl-4 benzènesulfonyl) aniline. Dichlorhydrate 185-187 (acétone)

Dichloro-2,6 N-[((chloro-4 phényl)-4 pipérazinyl-1)-3 hydroxy-2 propyl] N-(méthyl-4 benzènesulfonyl) aniline. Oxalate acide 152-155 (acétone)

Dichloro-2,6 N-[((chloro-3 phényl)-4 pipérazinyl-1)-3 hydroxy-2 propyl] N-(méthyl-4 benzènesulfonyl) aniline. Oxalate acide 178-179,5 (acétone)

Dichloro-2,6 N-[((fluoro-4 phényl)-4 pipérazinyl-1)-3 hydroxy-2 propyl] N-(méthyl-4 benzènesulfonyl) aniline. Oxalate acide 167-169 (acétone)

Dichloro-2,6 N-[hydroxy-2((méthoxy-2 phényl-4) pipérazinyl-1)-3 propyl] N-méthyl-4 benzènesulfonyl) aniline. Oxalate acide 192-195 (acétone)

Dichloro-3,4 N-[hydroxy-2(méthyl-4 pipérazinyl-1)-3 propyl] N-méthylsulfonyl aniline. Dichlorhydrate 224 (éthanol)

Dichloro-3,4 N-[((chloro-2 phényl)-4 pipérazinyl-1)-3 hydroxy-2 propyl] N-méthylsulfonyl aniline. Chlorhydrate 115-117 (éthanol)

Dichloro-3,4 N-[((chloro-2 phényl)-4 pipérazinyl-1)-3 hydroxy-2 propyl] N-méthylsulfonyl aniline. Dichlorhydrate 186 (éthanol)

Dichloro-3,4 N-[hydroxy-2((méthoxy-2 phényl)-4 pipérazinyl-1)-3 propyl] N-méthylsulfonyl aniline. Dichlorhydrate 209-210 (isopropanol)

Dichloro-3,4 N-[hydroxy-2(méthoxy-4 phényl)-4 pipérazinyl-1)-3 propyl] N-méthylsulfonyl aniline. Dichlorhydrate 195 (éthanol)

Dichloro-3,4 N-[hydroxy-2(phényl-4 pipérazinyl-1)-3 propyl] N-méthylsulfonyl aniline 107-108 (éthanol)

Dichloro-3,4 N-[hydroxy-2(méthyl-4 pipérazinyl-1)-3 propyl] N-(méthyl-4 benzènesulfonyl) aniline. Dichlorhydrate 250 (isopropanol)

Dichloro-3,4 N-[hydroxy-2(phényl-4 pipérazinyl-1)-3 propyl] N-(méthyl-4 benzènesulfonyl) aniline 163-164 (isopropanol)

Dichloro-3,4 N-[hydroxy-2(méthyl-4 pipérazinyl-1)-3 propyl] N-(méthoxy-4

benzènesulfonyl) aniline. Dichlorhydrate 237-238 (isopropanol)

Dichloro-3,4 N-[hydroxy-2(méthyl-4 pipérazinyl-1)-3 propyl] N-(méthoxy-4 benzènesulfonyl) aniline 152-153 (éthanol)

Dichloro-3,4 N-[((chloro-4 phényl)-4 pipérazinyl-1)-3 hydroxy-2 propyl] N-(méthoxy-4 benzènesulfonyl) aniline. Dichlorhydrate 164-165 (isopropanol)

Dichloro-3,4 N-[hydroxy-2((méthoxy-4 phényl)-4 pipérazinyl-1)-3 propyl] N-(méthoxy-4 benzènesulfonyl) aniline. Dichlorhydrate 181-182 (éthanol)

Acétylamino-4 N-[hydroxy-2(méthyl-4 pipérazinyl-1)-3 propyl] N-méthylsulfonyl aniline. Dichlorhydrate 205-208 (méthanol)

Acétylamino-4 N-[((chloro-4 phényl)-4 pipérazinyl-1)-3 hydroxy-2 propyl] N-méthylsulfonyl aniline 167-168 (éthanol)

Acétylamino-4 N-[hydroxy-2(phényl-4 pipérazinyl-1)-3 propyl] N-méthylsulfonyl aniline 177-179 (méthanol)

Acétylamino-4 N-[hydroxy-2((méthoxy-4 phényl)-4 pipérazinyl-1)-3 propyl] N-méthylsulfonyl aniline 195-197 (éthanol)

Acétylamino-4 N-[hydroxy-2((méthoxy-2 phényl)-4 pipérazinyl-1)-3 propyl] N-méthylsulfonyl aniline 130-133 (éthanol)

Acétylamino-4 N-[((chloro-2 phényl)-4 pipérazinyl-1)-3 hydroxy-2 propyl] N-méthylsulfonyl aniline 142-144 (isopropanol)

Acétylamino-4 N-[hydroxy-2(phényl-4 pipérazinyl-1)-3 propyl] N-méthyl-4 benzènesulfonyl aniline 175-177 (éthanol)

Acétylamino-4 N-[hydroxy-2((méthoxy-4 phényl)-4 pipérazinyl-1)-3 propyl] N-(méthyl-4 benzènesulfonyl) aniline. Dichlorhydrate 158-162 (méthanol)

Acétylamino-4 N-[hydroxy-2((méthoxy-2 phényl)-4 pipérazinyl-1)-3 propyl] N-(méthyl-4 benzènesulfonyl) aniline. Chlorhydrate 137-142 (éthanol)

Acétylamino-4 N-[((chloro-2 phényl)-4 pipérazinyl-1)-3 hydroxy-2 propyl] N-(méthyl-4 benzènesulfonyl) aniline. Chlorhydrate 170-172 (éthanol)

Acétylamino-4 N-[((chloro-4 phényl)-4 pipérazinyl-1)-3 hydroxy-2 propyl] N-(méthyl-4 benzènesulfonyl) aniline 177-178 (éthanol)

N-[hydroxy-2(méthyl-4 pipérazinyl-1)-3 propyl] N-méthylsulfonyl amino-4 phénylacétamide 129-135 (isopropanol/éther)

N-[hydroxy-2(phényl-4 pipérazinyl)-1)-3 propyl] N-méthylsulfonyl amino-4 phénylacétamide 155-157 (éthanol)

N-[((chloro-4 phényl)-4 pipérazinyl-1)-3 hydroxy-2 propyl] N-méthylsulfonyl amino-4 phénylacétamide 172-173 (éthanol)

N-[((chloro-2 phényl)-4 pipérazinyl-1)-3 hydroxy-2 propyl] N-méthylsulfonyl amino-4 phénylacétamide 177-178 (éthanol)

N-[hydroxy-2((méthoxy-4 phényl)-4 pipérazinyl-1)-3 propyl] N-méthylsulfonyl amino-4 phénylacétamide 171-172 (éthanol)

N-[hydroxy-2((méthoxy-2 phényl)-4 pipérazinyl-1)-3 propyl] N-méthylsulfonyl amino-4 phénylacétamide 195-197 (éthanol)

N-[hydroxy-2(méthyl-4 pipérazinyl-1)-3 propyl] N-(méthyl-4 benzènesulfonyl) amino-4 phénylacétamide. Dichlorhydrate 217-219 (éthanol)

N-[hydroxy-2(phényl-4 pipérazinyl-1)-3 propyl] N-(méthyl-4 benzènesulfonyl)

amino-4 phénylacétamide 158-161 (éthanol)

N-[hydroxy-2((méthoxy-2 phényl)-4 pipérazino-1)-3 propyl] N-(méthyl-4 benzènesulfonyl) amino-4 phénylacétamide 145-147 (éthanol)

N-[((chloro-4 phényl)-4 pipérazinyl-1)-3 hydroxy-2 propyl] N-(méthyl-4 benzènesulfonyl) amino-4 phénylacétamide 177-179 (éthanol)

N-[((chloro-2 phényl)-4 pipérazinyl-1)-3 hydroxy-2 propyl] N-(méthyl-4 benzènesulfonyl) amino-4 phénylacétamide 160-161 (éthanol)

N-[hydroxy-2 ((méthoxy-4 phényl)-4 pipérazino-1)-3 propyl] N-(méthyl-4 benzènesulfonyl amino-4 phénylacétamide 157-158 (éthanol)

On a enfin préparé, selon des techniques pharmaceutiques bien connues, des compositions telles que :

1. Gélule

| Ingrédients | mg |
|---|---|
| Chlorhydrate d'allyloxy-2 N-[tert-butylamino-3 hydroxy-2 propyl] N-(méthoxy-4 benzènesulfonyl) aniline | 100 |
| Amidons | 99,5 |
| Silice colloïdale | 0,5 |
| | 200,0 |

2. Solution injectable

| Ingrédients | mg |
|---|---|
| Dichlorhydrate d'allyloxy-2 N-[(benzyl-4 pipérazinyl-1)-3 hydroxy-2 propyl] N-(méthyl-4 benzènesulfonyl) aniline | 150 |
| Polysorbate 80 | 150 |
| Alcool benzylique | 75 |
| Eau | q.s. 3 ml |

3. Suppositoire

| Ingrédients | mg |
|---|---|
| Chlorhydrate d'allyloxy-2 N-[hydroxy-2 ((méthoxy-2 phényl)-4 pipérazinyl-1)-3 propyl] N-méthylsulfonyl aniline | 100 |
| Mélange de mono- et diglycérides d'acides saturés ($C_{12}$ à $C_{18}$) | 1 400 |
| | 1 500 |

4. Comprimés

| Ingrédients | mg |
|---|---|
| Acétylamino-4 N-[hydroxy-2 (phényl-4 pipérazinyl-1)-3 propyl] N-méthylsulfonyl aniline | 200 |
| Lactose | 64 |
| Polyvinylpyrrolidone | 6 |
| Carboxylate d'amidon sodique | 24 |
| Stéarate de magnésium | 4 |
| Talc | 2 |
| | 300 |

**Revendications**

1. Nouveau composé de formule générale :

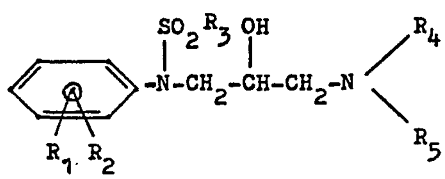

dans laquelle $R_1$ et $R_2$, qui sont identiques ou différents, représentent un groupement allyloxy, acétamido ou carboxamido, ou un atome d'hydrogène ou de chlore, $R_3$ représente un groupement méthyle, phényle, méthylphényle ou méthoxyphényle, $R_4$ représente un atome d'hydrogène et $R_5$ un groupement isopropyle, terbutyle, phénoxy-2 éthyle, phényl-3 propyle, ou $R_4$ et $R_5$ forment avec l'atome d'azote un hétérocycle tel que pyrrolidine, morpholine ou une pipérazine substituée de formule générale :

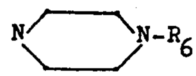

II

dans laquelle $R_6$ représente un groupement alkyle linéaire contenant de 1 à 3 atomes de carbone, un radical hydroxy-2 éthyle, phényle, phényle substitué par un atome d'halogène ou par un groupement méthoxy, le groupement benzyle ou pyridyle-2, ou un de ses sels d'addition d'acide pharmaceutiquement acceptables.

2. Allyloxy-2 N-[tert. butylamino-3 hydroxy-2 propyl] N-(méthoxy-4 benzènesulfonyl) aniline.

3. Allyloxy-2 N-[hydroxy-2 ((méthoxy-2 phényl)-4 pipérazinyl-1)-3 propyl] N-méthylsulfonyl aniline.

4. Allyloxy-2 N-[(benzyl-4 pipérazinyl-1)-3 hydroxy-2 propyl] N-(méthyl-4 benzènesulfonyl) aniline.

5. Acétylamino-4 N-[hydroxy-2 (phényl-4 pipérazinyl-1)-3 propyl] N-méthylsulfonyl aniline.

6. Procédé de préparation d'un composé selon la Revendication 1 caractérisé en ce que l'on fait réagir une amine de formule générale :

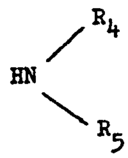

III

dans laquelle $R_4$ et $R_5$ ont les mêmes significations que dans la Revendication 1, avec un composé de formule générale :

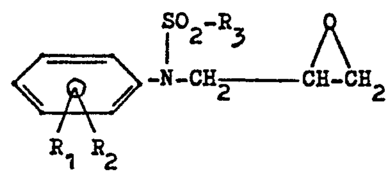

IV

dans laquelle $R_1$, $R_2$ et $R_3$ ont les mêmes significations que dans la Revendication 1.

7. Procédé selon la Revendication 6 caractérisé en ce que la réaction s'effectue dans un solvant alcoolique tel que l'éthanol.

8. Nouveau médicament constitué par au moins un composé de formule I ou un de ses sels d'addition d'acide pharmaceutiquement acceptable.

9. Composition pharmaceutique contenant comme principe actif au moins un composé de formule I, ou un de ses sels d'addition d'acide pharmaceutiquement acceptable, en association avec un excipient pharmaceutique.

10. Composition selon la Revendication 9 caractérisée en ce qu'elle se présente sous une forme convenant à l'administration orale ou rectale.

11. Composition selon la Revendication 9 caractérisée en ce qu'elle se présente sous la forme d'une solution convenant pour l'administration par injection.

12. Composés selon la Revendication 1 utiles comme agents thérapeutiques pour le traitement de l'angine de poitrine.

13. Composés selon la Revendication 1 utiles à la dose journalière de 100 à 200 mg comme agents thérapeutiques pour le traitement de l'angine de poitrine.

**Claims**

1. New compound of the general formula :

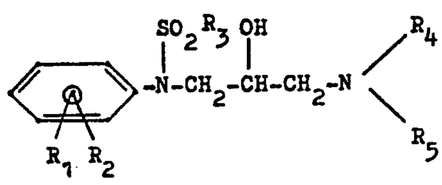

and the pharmaceutically acceptable acid addition salts thereof, wherein $R_1$ and $R_2$, which are the same or different, represent an allyloxy, acetamide or carboxamide group or a hydrogen or chlorine atom, $R_3$ represents a methyl, phenyl, methylphenyl or methoxyphenyl group, $R_4$ represents a hydrogen atom and $R_5$ an isopropyl, terbutyl, 2-phenoxy-ethyl, 3-phenyl-propyl group, or $R_4$ and $R_5$ taken together form with the nitrogen atom a heterocycle such as pyrrolidine, morpholine or a substituted piperazine of the formula :

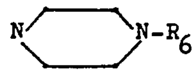

II

wherein $R_6$ represents a straight-chain alkyl group containing from 1 to 3 carbon atoms, a 2-hydroxyethyl, a benzyl or a 2-pyridyl group or a phenyl group optionally substituted by either a halogen atom or by a methoxy radical.

2. 2-Allyloxy-N-(3-tert-butylamino-2-hydroxy-propyl)-N-(4-methoxybenzenesulphonyl)-aniline.

3. 2-Allyloxy-N-[2-hydroxy-3-(4-(2-methoxy-phenyl)-1-piperazinyl)-propyl]-N-methylsulphonyl-aniline.

4. 2-Allyloxy-N-[3-(4-benzyl-1-piperazinyl)-2-hydroxy-propyl]-N-(4-methyl-benzenesulphonyl)-aniline.

5. 4-Acetylamino-N-[2-hydroxy-3-(4-phenyl-1-piperazinyl)-propyl]-N-methylsulphonyl-aniline.

6. Process for preparing a compound according to Claim 1 whereby an amine of general formula :

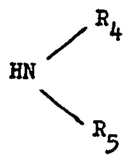

III

wherein $R_4$ and $R_5$ have the same meanings as in Claim 1, is reacted with a compound of general formula :

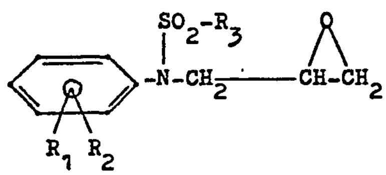

IV

wherein $R_1$, $R_2$ and $R_3$ have the same meanings as in Claim 1.

7. Process according to Claim 6 whereby the reaction is carried out in an alcoholic solvent such as ethanol.

8. Novel medicament comprising at least one compound of formula I, or a pharmaceutically acceptable acid addition salt thereof.

9. Pharmaceutical composition containing as active ingredient at least one compound of formula I, or a pharmaceutically acceptable acid addition salt thereof, together with an appropriate excipient or carrier therefor.

10. Pharmaceutical composition according to Claim 9 for oral or rectal administration.

11. Pharmaceutical composition according to Claim 9 in the form of an injectable solution.

12. Compounds according to Claim 1 for use as therapeutic agents for the treatment of angina pectoris.

13. Compounds according to Claim 1 for use, at a daily dosage of 100 to 200 mg, as therapeutic agents for the treatment of angina pectoris.

**Ansprüche**

1. Neue Verbindung der allgemeinen Formel :

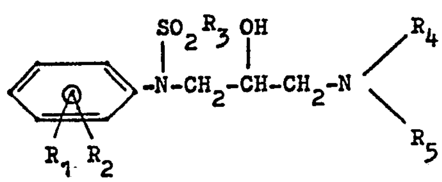

und die pharmazeutisch verträglichen Säureadditionssalze davon, wobei $R_1$ und $R_2$, die gleich oder

verschieden sind, eine Allyloxy-, Acetamid- oder Carboxamidgruppe oder ein Wasserstoff- oder Chloratom darstellen, $R_3$ eine Methyl-, Phenyl-, Methylphenyl- oder Methoxyphenylgruppe darstellt, $R_4$ ein Wasserstoffatom darstellt und $R_5$ eine Isopropyl-, tert-Butyl-, 2-Phenoxyethyl- oder 3-Phenyl-propyl-Gruppe oder $R_4$ und $R_5$ zusammen mit dem Stickstoffatom einen Heterocyclus bilden, z. B. Pyrrolidin, Morpholin oder ein substituiertes Piperazin der Formel :

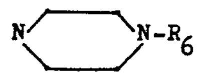

II

worin $R_6$ eine geradkettige Alkylgruppe mit 1 bis 3 Kohlenstoffatomen, eine 2-Hydroxyethyl-, Benzyl- oder 2-Pyridylgruppe oder eine Phenylgruppe darstellt, die gegebenenfalls entweder durch ein Halogenatom oder einen Methoxyrest substituiert ist.

2. 2-Allyloxy-N-(3-tert-butylamino-2-hydroxy-propyl)-N-(4-methoxy-benzolsulfonyl)-anilin.

3. 2-Allyloxy-N-[2-hydroxy-3-(4-(2-methoxy-phenyl)-1-piperazinyl)-propyl]-N-methylsulfonyl-anilin.

4. 2-Allyloxy-N-[3-(4-benzyl-1-piperazinyl)-2-hydroxypropyl]-N-(4-methyl-benzosulfonyl)-anilin.

5. 4-Acetylamino-N-[2-hydroxy-3-(4-phenyl-1-piperazinyl)-propyl]-N-methylsulfonyl-anilin.

6. Verfahren zur Herstellung einer Verbindung nach Anspruch 1, in dem ein Amin der allgemeinen Formel :

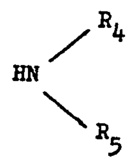

III

worin $R_4$ und $R_5$ dieselben Bedeutungen wie in Anspruch 1 haben, mit einer Verbindung der allgemeinen Formel :

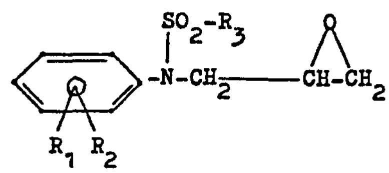

IV

worin $R_1$, $R_2$ und $R_3$ dieselben Bedeutungen wie in Anspruch 1 haben, umgesetzt wird.

7. Verfahren nach Anspruch 6, bei dem die Reaktion in einem alkoholischen Lösungsmittel, wie Ethanol, durchgeführt wird.

8. Neuartiges Medikament, umfassend mindestens eine Verbindung der Formel I oder ein pharmazeutisch verträgliches Säureadditionssalz davon.

9. Pharmazeutische Zusammensetzung, enthaltend als Wirkstoff mindestens eine Verbindung der Formel I oder ein pharmazeutisch verträgliches Säureadditionssalz davon zusammen mit einem geeigneten Excipienten oder Träger dafür.

10. Pharmazeutische Zusammensetzung nach Anspruch 9 für die orale oder rektale Applikation.

11. Pharmazeutische Zusammensetzung nach Anspruch 9 in Form einer injizierbaren Lösung.

12. Verbindungen nach Anspruch 1 zur Verwendung als therapeutische Mittel zur Behandlung von Angina pectoris.

13. Verbindungen nach Anspruch 1 zur Verwendung in einer Tagesdosis von 100 bis 200 mg als therapeutische Mittel zur Behandlung von Angina pectoris.